# EUROPÄISCHE PATENTSCHRIFT

(11) **EP 1 087 921 B1**
(45) Veröffentlichungstag und Bekanntmachung des Hinweises auf die Patenterteilung: **18.02.2004**
(21) Anmeldenummer: 99926495.5
(22) Anmeldetag: 01.06.1999
(51) Int. Cl.: C07C 29/94, C07C 41/46

(54) **ALTERUNGSSCHUTZMITTEL FÜR GLYKOLE ODER GLYKOLETHER**
ANTI-AGING AGENT FOR GLYCOLS OR GLYCOL ETHERS
AGENT ANTI-VIEILLISSEMENT POUR GLYCOLS OU GLYCOLETHERS

(30) Priorität: 17.06.1998 DE 19826914
(43) Veröffentlichungstag der Anmeldung: 04.04.2001
(73) Patentinhaber: RUHRGAS AKTIENGESELLSCHAFT, 45138 Essen (DE)
(72) Erfinder: FORSTER, Ruediger, D-45894 Gelsenkirchen (DE); JOOS, Katharina, D-46282 Dorsten (DE)
(74) Vertreter: Harlacher, Mechthild
(86) Internationale Anmeldenummer: PCT/EP1999/003782
(87) Internationale Veröffentlichungsnummer: WO 1999/065850

(56) Entgegenhaltungen:
- DE-A- 19 602 116
- DE-B- 1 242 586
- CHEMICAL ABSTRACTS, vol. 81, no. 23, 9. Dezember 1974 (1974-12-09) Columbus, Ohio, US; abstract no. 151536c, Seite 476; XP002029878 & JP 49 051212 A (MITSUI PETROCHEMICAL INDUSTRIES) 18. Mai 1974 (1974-05-18)

## Beschreibung

Die Erfindung betrifft ein Additiv für ein Glykol oder einen Glykolether zur Erhöhung der Alterungsbeständigkeit. Glykole, insbesondere Triethylenglykol, werden unter anderem in Erdgastrocknungsanlagen eingesetzt. In diesen Anlagen werden sie in einem Kreislauf geführt, wobei sie in einem Absorber Feuchtigkeit aus einem zu trocknenden Gas aufnehmen und anschließend in einem Reboiler thermisch regeneriert werden. Das Glykol ist dabei Temperaturen bis ca. 220 °C ausgesetzt. Durch die hohe thermische Beanspruchung kommt es zu einer Alterung des Glykols. Die Alterung ist auf einen thermischen Zerfall bzw. einen thermooxidativen Abbau zurückzuführen. Zusätzlich treten Reaktionen mit Stoffen auf, die aus dem zu trocknenden Gas aufgenommen wurden. Durch Anlagenkorrosion gebildete Metallionen beschleunigen die Glykolalterung.

Die thermoxidative Alterung findet bei Temperaturen oberhalb von etwa 130 °C in Gegenwart von Sauerstoff statt. Durch einen Radikalkettenmechanismus wird das Glykol zersetzt. Primär entstehen durch den Sauerstoffangriff Hydroperoxide, die rasch weiter zerfallen. Dabei werden Aldehyde, Carbonsäuren, Ester und Ether gebildet.
Die Hitzealterung beruht auf einem oft als "Cracken" bezeichneten thermischen Abbau des Glykols. Wie bei dem zuvor genannten sauerstoffinduzierten Mechanismus handelt es sich um eine Radikalreaktion, bei der eine C-O-Etherbindung des Glykols homolytisch gespalten wird. Die dabei entstehenden stabilen Reaktionsprodukte sind denen der Thermoxidation sehr ähnlich.

Metallionen katalysieren die Alterung. Schädlich ist insbesondere Eisen, das durch Anlagenkorrosion in das Glykol gelangt. Durch die metallionenkatalysierte Alterung können unter anderem Fettalkohol-Ethoxylate und Alkylaromaten, wie z. B. Alkylphenol-Ethoxylate, gebildet werden.

Die so gebildeten Verunreinigungen des Glykols mindern dessen Wirksamkeit bei der Trocknung und führen zu unerwünschten Nebeneffekten, wie beispielsweise dem Glykolschäumen. Dann werden eine aufwendige Reinigung oder ein Austausch des Glykols erforderlich, was die Kosten des Betriebs der Trocknungsanlage beträchtlich erhöht. Glykole oder Glykolether werden ferner in Trocknungs- und Reinigungsprozessen von Synthese-, Spalt-, sowie Raffineriegasen eingesetzt.

Zu den Glykolethern sind sowohl Diethylenglykol, Triethylenglykol als auch Polyalkylenglykole (auch als Polyglykole bezeichnet) zu rechnen. Die Polyalkylenglykole umfassen u. a. die Polyethylenglykole (auch als Polyethylenoxide bezeichnet) und die Carbowachse. Mit aliphatischen Alkoholen oder Phenolen veretherte Polyglykole (ebenfalls als Glykolether bezeichnet) spielen eine wichtige Rolle als Wärmeüberträgerflüssigkeiten. Eine Reihe dieser Glykolether bildet Bestandteile einer großen Anzahl chemischer Produkte. Sowohl bei der oben beschriebenen Verwendung der Glykole oder Glykolether in Trocknungsanlagen, als auch bei deren Verwendung als Bestandteil chemischer Produkte kommt der Alterungsbeständigkeit eine hohe Bedeutung zu.

Zur Erhöhung der Alterungsbeständigkeit werden den Glykolen bzw. Glykolethern Additive beigemischt, die den einzelnen Alterungsmechanismen entgegenwirken. Diese als Alterungsschutzmittel verwendeten Additive umfassen Antioxidantien, die in der Regel ein Gemisch verschiedener Stoffkomponenten darstellen, die sich in ihrer Wirkung gegenseitig unterstützen (Synergismen). Diese Stoffkomponenten umfassen Radikalfänger, Peroxidzersetzer, Synergisten und Metalldesaktivatoren. Als Antioxidantien verwendete Stoffe umfassen beispielsweise durch sterisch hindernde Gruppen substituierte Phenole und aromatische Amine (vergleiche Römpp, Chemie-Lexikon, 9. Auflage, 1995, Seite 220).

Die Verwendung aromatischer Amine als Additive für Glykole ist beispielsweise aus R. S. Googlev, M. B. Neimann, "Thermal-Oxidative Degradation of the Simpler Polyalkyleneoxides", Polymer Science USSR 9 (1967), Nr. 10, S. 2351 bekannt. Auch die Kombination eines phenolischen Antioxidans (2,6-Di-tert.-butyl-phenol) und eines aminischen Antioxidans zur Alterungsstabilisierung von Polyglykolen ist bekannt (beispielsweise aus einer Firmenbroschüre der Rhein Chemie Rheinau GmbH, 1995).

Aus dem Chemical Abstracts Bd. 81, No. 23 vom 9. Dezember 1974, PX 002029878, Seite 476 ist die Zugabe eines organischen Phosphits oder Phosphats zu einer Glykolmischung bei deren Reinigung durch Destillation bekannt.

Aufgabe der Erfindung ist es, den Alterungsschutz für ein Glykol oder Glykol-ether insbesondere bei höheren Temperaturen zu verbessern.

Diese Aufgabe wird bei dem Einsatz eines Glykols in einer Gastrocknungsanlage erfindungsgemäß durch die Verwendung eines organischen Phosphonats nach Patentanspruch 1 gelöst.

Femer wird die Aufgabe durch ein Additiv mit den Merkmalen des Patentanspruchs 5, ein Additiv mit den Merkmalen des Patentanspruchs 11 sowie ein Additiv mit den Merkmalen des Patentanspruchs 24 gelöst.

Die Verwendung eines organischen Phosphonats (Esters der Phosphonsäure) als Additiv für ein in einer Gastrocknungsanlage umlaufendes Glykol, hat einen positiven Einfluß auf die Alterungsbeständigkeit. Die thermisch stabilen und hydrolyseunempfindlichen organischen Phosphonate, insbesondere die Hydroxy-phosphono-Carbonsäuren, widerstehen den Prozeßbedingungen gut. Sie wirken als Korrosionsinhibitoren. Außerdem verhindern sie als Scale-Inhibitoren die Ablagerung von Kalziumchlorid im Glykolkreislauf. Weiterhin haben sie einen günstigen Einfluß auf das Emulgierverhalten des Glykols, da sie durch Komplexbildung (positive) Oberflächenladungen dispergierter Partikel neutralisieren können und damit eine Dispersion destabilisieren. Kationische Oberflächenladungen können durch Metallionen oder die aus Alkanolaminen, welche üblicherweise zur pH-Wert-Stabilisierung von Glykol eingesetzt werden, gebildeten sogenannten thermostabilen Salze hervorgerufen werden. Die genannte Wirkung tritt vor allem bei niedriger Konzentration der Phosphonate bzw. Phosphate auf. Das Additiv wird in einer Menge zwischen 0,01 und 0,1 Gew.-% vorzugsweise 0,02 - 0,05 Gew.-%, zugegeben.

Bei einer bevorzugten Ausführungsform wird das Phosphonat vor dem Einsatz als Additiv im Prozeß einem Glykol, vorzugsweise Triethylenglykol, in einer Menge zwischen 1 und 5 Gew.-%, vorzugsweise 2 - 5 Gew.-%, zugemischt wird und diese Mischung dem in der Gastrocknungsanlage umlaufenden Glykol in einer Konzentration von ca. 1 Gew.-% zugegeben.

Eine Ausführungsform des erfindungsgemäßen Additivs für ein Glykol oder einen Glykolether weist einen Ester des Glycerins mit wenigstens einer eine sterisch gehinderte Hydroxyphenyl-Gruppe enthaltenden linearen Carbonsäure sowie einer Hydroxy-Phosphono-Carbonsäure auf. Die die sterisch gehinderte Hydroxyphenyl-Gruppe aufweisende Verbindung wird auch als sterisch gehindertes Phenol bezeichnet. Durch die Kombination des Esters mit der. Hydroxy-Phosphono-Carbonsäure tritt ein synergistischer Effekt auf, wobei sich die Wirkungen der Stoffe verstärken. Diese synergistischen Effekte verbessern den Hitzealterungs- und Oxidationsschutz des Glykols oder Glykolethers. Die verbesserte Antioxidans-Wirkung beruht unter anderem auf dem Synergismus von peroxidzersetzender Wirkung der Hydroxy-Phosphono-Carbonsäure und Radikalfänger-Wirkung des sterisch gehinderten Phenols. Es zeigte sich ferner eine verringerte Tendenz zur Schlammbildung durch schwerlösliche Alterungsprodukte, eine verminderte Bildung von emulgierend wirkenden Alterungsprodukten, die ansonsten zur verstärkten Aufnahme von Partikeln und Fetten durch das Glykol führen, und eine geringere Tendenz zur Verfärbung des Glykols durch "Crack"-Produkte.

Bei einer zweiten Ausführungsform weist das Additiv erstens eine eine sterisch gehinderte Hydroxyphenyl-Gruppe aufweisende Verbindung, zweitens eine Hydroxy-Phosphono-Carbonsäure und drittens ein aromatisches aminisches Antioxidans auf. Eine sterisch gehinderte Hydroxyphenyl-Gruppe aufweisende Verbindungen sind als.phenolische Antioxidantien bekannt. Ebenso ist deren Kombination und Synergismus mit aminischen Antioxidantien bekannt. Durch die Zumischung einer Hydroxy-Phosphono-Carbonsäure wird bei der vorliegenden Erfindung dieser Synergismus verstärkt und durch zusätzliche positive Einflüsse der Phosphonsäure ergänzt.

Bei beiden genannten Ausführungsformen des Additivs können die eine sterisch gehinderte Hydroxyphenyl-Gruppe aufweisende Verbindung und die Hydroxy-Phosphono-Carbonsäure aneinander gebunden sein, beispielsweise durch Veresterung einer Hydroxy-Phosphono-Carbonsäure mit einem partiellen Mischester des Glycerins mit einer die sterisch gehinderte Hydroxyphenyl-Gruppe aufweisenden linearen Carbonsäure und einer weiteren linearen Carbonsäure.

In Weiterbildung der Erfindung enthält das Additiv eine Polycarbonsäure. Diese steigert die auf der Phosphonsäure beruhende Korrosionschutzwirkung des Additivs. Als Polycarbonsäure kommt zunächst jede Verbindung mit mehreren Carboxyl-Gruppen in Betracht. Vorzugsweise wird jedoch eine stickstoffhaltige Polycarbonsäure verwendet, bei der Carbonsäuren über Imino-Gruppen an ein Derivat des Triazins gebunden sind.

Eine vorteilhafte Weiterbildung der Erfindung ist durch die Zugabe eines Alkanolamins, insbesondere eines tertiären Alkanolamins, gekennzeichnet. Bevorzugt werden Triethanolamin und Methyldiethanolamin. Sie haben den Vorteil der geringeren Flüchtigkeit und zeigen weniger Nebenreaktionen als Monooder Diethanolamin. Durch die Kombination der Phosphonsäureverbindung mit dem Alkanolamin findet eine Verringerung des Korrosionsangriffs auf Stahl auch bei hohen Temperaturen statt. Es wird auf dem Stahl eine Passivschicht von Metallphosphonaten gebildet, wodurch die weitere Anlagenkorrosion gemindert wird. Es verringert sich die durch die Korrosion aufgelöste Eisenmenge. Das gelöste Eisen, das auf die Glykolzersetzung katalytisch wirkt, wird durch Komplexbildung desaktiviert; es bilden sich stabile Komplexe der Lewis-Säure Eisen mit der Lewis-Base Phosphonsäure.

Eine dritte Ausführungsform des erfindungsgemäßen Additivs für ein Glykol oder ein Glykolether weist eine Hydroxy-Phosphono-Carbonsäure und ein tertiäres Alkanolamin auf. Bereits die Kombination dieser beiden Stoffe verringert die metallionenkatalysierte Alterung des Glykols, insbesondere durch die Lewis-Säure Eisen. Dies beruht auf der oben angegebenen Bildung stabiler Komplexe. Als tertiäres Alkanolamin werden (aus den oben genannten Gründen) vorzugsweise Triethanolamin und/oder Methyldiethanolamin verwendet.

Eine vorteilhafte Weiterbildung dieses Additivs ist durch die Zugabe einer Polycarbonsäure gekennzeichnet, wobei vorzugsweise eine Verbindung verwendet wird, bei der Carbonsäuren über imino-Gruppen an ein Derivat des Triazins gebunden sind.

Weitere vorteilhafte Weiterbildungen der Erfindung sind in den Unteransprüchen gekennzeichnet.

im folgenden wird die Erfindung anhand eines bevorzugten Ausführungsbeispiels näher beschrieben.

Das als Additiv dem Glykol oder Glykolether, vorzugsweise dem Triethylenglykol in einer Gastrocknungsanlage, zugegebene Alterungsschutzmittel stellt eine Mischung eines eine sterisch gehinderte Hydroxyphenyl-Gruppe aufweisenden phenolischen Antioxidans, eines aminischen Antioxidans, einer Phosphono-Carbonsäure, .einer Polycarbonsäure und eines tertiären Alkanolamins in Triethylenglykol dar. Als besonders wirkungsvoll hat sich die in der folgenden Tabelle dargestellten Rezeptur herausgestellt:

| **Additiv-Komponente** | **Gewichtsanteile in %** |
|---|---|
| Hydroxy-Phosphono-Carbonsäure (BELCOR 575 der FMC Corp. (USA, UK)) | 1-5; vorzugsweise 2,5 - 5 |
| sterisch gehinderte Phenolalkylen-Carbonsäure-Mischester mit C8-C18 linearen Fettsäuren und Glycerin (Irgalube F10 der Fa. CIBA Spezialitätenchemie) | 0,01 - 2; vorzugsweise 0,1 - 0,5 |
| N-Phenylbenzolamin, styrolisiert (Additin RC 7135, Fa. Rhein-Chemie) | 0,01 - 2; vorzugsweise 0,1 - 0,5 |
| Triethanolamin oder Methyldiethanolamin | 5 - 25; vorzugsweise 5 - 15 |
| 6, 6', 6"-(1,3,5-triazin-2,4,6-triyltriimino)-trihexansäure (Irgacor L 190, Fa. CIBA Spezialitätenchemie) | 1-10; vorzugsweise 2,5 - 5 |
| Triethylenglykol | Rest |

Die in der Tabelle angegebene Mischung wird dem zu schützenden Glykol einer Gastrocknungsanlage in einer Konzentration von etwa 1 % zugesetzt. Alternativ kann die Mischung vorab auch mit einer geringeren oder höheren Konzentration des Triethylenglykols oder auch eines anderen Glykols hergestellt werden. Für die Wirkung des Alterungsschutzmittels ist letztendlich nur die Konzentration in dem im Prozeß eingesetzten zu schützenden Glykol von Bedeutung. Die Vorab-Mischung mit einem Glykol dient vorrangig der besseren Handhabung und Lagerstabilität des Additivs.

Zur Verbesserung der Lagerstabilität kann anstelle der Triazintricarbonsäure auch deren Alkalisalz eingesetzt werden. Dadurch kann eine zu hohe Beimengung des Alkanolamins vermieden werden, die zu einer Verfärbung des Glykols führt.

Die bezeichnete Mischung zeichnet sich insbesondere dadurch aus, daß sie neben einer guten Schutzwirkung gegen die thermoxidative Zersetzung auch eine sehr geringe Tendenz zur Schlammbildung besitzt, die sich in einer geringeren Tendenz zur Schwarzfärbung und einer langsameren Viskositätserhöhung bei der Alterung des Glykols bemerkbar macht.

## Patentansprüche

1. Verwendung eines organischen Phosphonats als temperaturbeständiges Alterungsschutzmittel für ein in einer Gastrocknungsanlage umlaufendes Glykol, welches in einem Prozeß eingesetzt wird, bei dem bei weitgehendem Sauerstoffausschluß Temperaturen bis ca. 220 C auftreten, wobei das Phosphonat in einer Menge zwischen 0,01 und 0,1 Gew.-%, vorzugsweise 0,02 - 0,05 Gew.-%, zugegeben wird.

2. Verfahren nach Anspruch 1, **dadurch gekennzeichnet, daß** das Phosphonat vor dem Einsatz als Additiv im Prozeß einem Glykol, vorzugsweise Triethylenglykol, in einer Menge zwischen 1 und 5 Gew.-%, vorzugsweise 2 - 5 Gew.-%, zugemischt wird und diese Mischung dem in der Gastrocknungsanlage umlaufenden Glykol in einer Konzentration von ca. 1 Gew.-% zugegeben wird.

3. Verfahren nach Anspruch 1, **dadurch gekennzeichnet, daß** als Phosphonat eine Hydroxy-Phosphono-Carbonsäure verwendet wird.

4. Verfahren nach Anspruch 3, **dadurch gekennzeichnet, daß** eine Hydroxy-Phosphono-Carbonsäure mit der Struktur 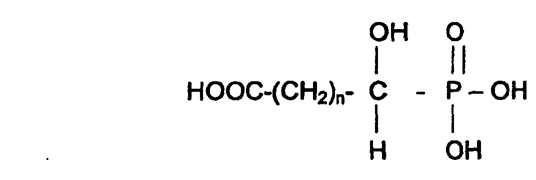 verwendet wird, wobei n = 0 .... 8 ist.

5. Additiv für ein Glykol oder ein Glykolether aufweisend:
einen Ester des Glycerins mit wenigstens einer eine sterisch gehinderte Hydroxyphenyl-Gruppe enthaltenden linearen Carbonsäure und
eine Hydroxy-Phosphono-Carbonsäure.

6. Additiv nach Anspruch 5, **dadurch gekennzeichnet, daß** der Ester ein partieller Mischester des Glycerins mit der wenigstens einen eine sterisch gehinderte Hydroxyphenyl-Gruppe enthaltenden linearen Carbonsäure und wenigstens einer weiteren linearen Carbonsäure ist.

7. Additiv nach Anspruch 5, **dadurch gekennzeichnet, daß** die sterisch gehinderte Hydroxyphenyl-Gruppe in Para-Stellung an die mit dem Glycerin veresterte Carbonsäure gebunden ist.

8. Additiv nach Anspruch 5, **dadurch gekennzeichnet, daß** die die sterisch gehinderte Hydroxyphenyl-Gruppe aufweisende lineare Carbonsäure eine Kette von 8 bis 18 Kohlenstoffatomen aufweist.

9. Additiv nach Anspruch 6, **dadurch gekennzeichnet, daß** es zu mindestens 70 Gew.-% Triethylenglykol, den partiellen Mischester des Glycerins in einer Menge von 0,01 - 2 Gew.-%, vorzugsweise 0,1 - 0,5 Gew.-%, und die Hydroxy-Phosphono-Carbonsäure in einer Menge von 1 - 5 Gew.-%, vorzugsweise 2 - 5 Gew.-%, enthält.

10. Additiv nach Anspruch 5, **dadurch gekennzeichnet, daß** die Hydroxy-Phosphono-Carbonsäure an das Glycerin oder an eine mit dem Glycerin veresterte Fettsäure gebunden ist.

11. Additiv für ein Glykol und/oder ein Glykolether aufweisend:
eine sterisch gehinderte Hydroxyphenyl-Gruppe aufweisende Verbindung,
eine Hydroxy-Phosphono-Carbonsäure und
ein aromatisches aminisches Antioxidans.

12. Additiv nach Anspruch 11, **dadurch gekennzeichnet, daß** die eine sterisch gehinderte Hydroxyphenyl-Gruppe aufweisende Verbindung und die Hydroxy-Phosphono-Carbonsäure aneinander gebunden sind.

13. Additiv nach Anspruch 11, **dadurch gekennzeichnet, daß** die eine sterisch gehinderte Hydroxyphenyl-Gruppe aufweisende Verbindung ein Ester des Glycerins mit wenigstens einer die sterisch gehinderte Hydroxyphenyl-Gruppe aufweisenden linearen Carbonsäure ist, wobei die Hydroxyphenyl-Gruppe in Para-Stellung an die mit dem Glycerin veresterte Carbonsäure gebunden ist.

14. Additiv nach Anspruch 13, **dadurch gekennzeichnet, daß** die die sterisch gehinderte Hydroxyphenyl-Gruppe aufweisende lineare Carbonsäure eine Kette von 8 bis 18 Kohlenstoffatomen aufweist.

15. Additiv nach einem der Ansprüche 11 - 14, **dadurch gekennzeichnet, daß** die Hydroxy-Phosphono-Carbonsäure die Struktur 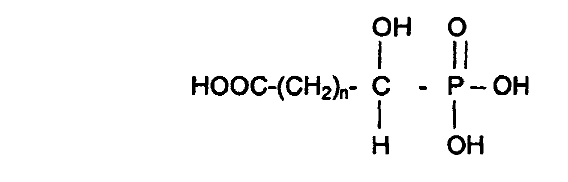 aufweist, wobei n = 0 ....8 ist.

16. Additiv nach Anspruch 11, **dadurch gekennzeichnet, daß** es zu mindestens 70 Gew.-% Triethylenglykol, die eine sterisch gehinderte Hydroxyphenyl-Gruppe aufweisende Verbindung in einer Menge von 0,01 - 2 Gew.-%, vorzugsweise 0,1 - 0,5 Gew.-%. die Hydroxy-Phosphono-Carbonsäure in einer Menge von 1 - 5 Gew.-%, vorzugsweise 2 - 5 Gew.-%, und das aromatische aminische Antioxidans in einer Menge von 0,01 - 2 Gew.-%, vorzugsweise 0,1 - 0,5 Gew.-%, enthält.

17. Additiv nach Anspruch 11, **dadurch gekennzeichnet, daß** es eine Polycarbonsäure enthält.

18. Additiv nach Anspruch 17, **dadurch gekennzeichnet, daß** es eine stickstoffhaltige Polycarbonsäure enthält.

19. Additiv nach Anspruch 18, **dadurch gekennzeichnet**, Polycarbonsäure wenigstens zwei über Imino-Gruppen an ein Derivat des Triazins bebundene Carbonsäuren enthält.

20. Additiv nach Anspruch 16, **dadurch gekennzeichnet, daß** es eine Polycarbonsäure in einer Menge von 1 - 10 Gew.-% vorzugsweise 2,5 - 5 Gew.-%, enthält.

21. Additiv nach Anspruch 20, **dadurch gekennzeichnet, daß** die Polycarbonsäure 6,6',6"-(1,3,5-triazin-2,4,6-triyltriimino)-trihexansäure aufweist.

22. Additiv nach Anspruch 11, **dadurch gekennzeichnet, daß** es ein Alkanolamin enthält.

23. Additiv nach Anspruch 22, **dadurch gekennzeichnet, daß** das Alkanolamin ein tertiäres Alkanolamin ist.

24. Additiv für ein Glykol oder ein Glykolether aufweisend:
eine Hydroxy-Phosphono-Carbonsäure und
ein tertiäres Alkanolamin.

25. Additiv nach Anspruch 24, **dadurch gekennzeichnet, daß** die Hydroxy-phosphono-Carbonsäure die Struktur 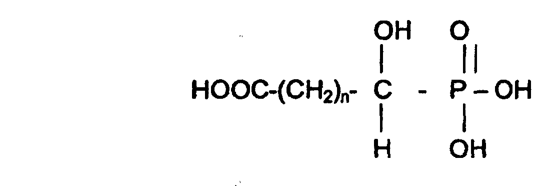 aufweist, wobei n = 0 ... 8 ist.

26. Additiv nach Anspruch 24, **dadurch gekennzeichnet, daß** das tertiäre Alkanolamin Triethanolamin und/oder Methyldiethanotamin umfaßt.

27. Additiv nach Anspruch 24, **dadurch gekennzeichnet, daß** es eine Polycarbonsäure enthält.

28. Additiv nach Anspruch 27, **dadurch gekennzeichnet, daß** es eine stickstoffhaltige Polycarbonsäure enthält.

29. Additiv nach Anspruch 28, **dadurch gekennzeichnet, daß** die Polycarbonsäure wenigstens zwei über Imino-Gruppen an ein Derivat des Triazins gebundene Carbonsäuren enthält.

## Claims

1. Use of an organic phosphonate as a temperature-resistant anti-aging agent for a glycol circulating in a gas drying unit, the glycol being used in a process in which temperatures of up to approx. 220°C occur under the virtual exclusion of oxygen, the phosphonate being added in an amount of between 0.01 and 0.1 weight %, preferably 0.02 - 0.05 weight %.

2. Process according to claim 1, **characterised in that** the phosphonate is added in an amount of between 1 and 5 weight %, preferably 2 to 5 weight %, to a glycol, preferably triethylene glycol, before use as an additive in the process, and that this mixture is added in a concentration of approx. 1 weight % to the glycol circulating in the gas drying unit.

3. Process according to claim 1, **characterised in that** a hydroxyphosphono carboxylic acid is used as the phosphonate.

4. Process according to claim 3, **characterised in that** a hydroxyphosphono carboxylic acid with the following structure 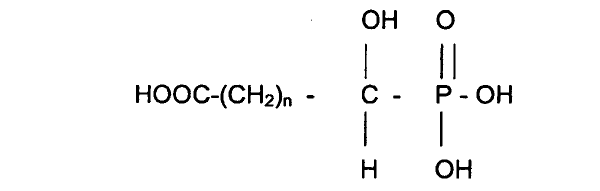 is used, where n = 0 ... 8.

5. Additive for a glycol or glycol ether exhibiting:
a glycerine ester with at least one linear carboxylic acid containing a sterically inhibited hydroxyphenyl group and
a hydroxyphosphono carboxylic acid.

6. Additive according to claim 5, **characterised in that** the ester is a partial mixed ester of glycerine with the at least one linear carboxylic acid containing a sterically inhibited hydroxyphenyl group and at least another linear carboxylic acid.

7. Additive according to claim 5, **characterised in that** the sterically inhibited hydroxyphenyl group is bonded in para position to the carboxylic acid esterified with the glycerine.

8. Additive according to claim 5, **characterised in that** the linear carboxylic acid exhibiting the sterically inhibited hydroxyphenyl group has a chain of 8 to 18 carbon atoms.

9. Additive according to claim 6, **characterised in that** it contains at least 70 weight % triethylene glycol, the partially mixed ester of glycerine in an amount of 0.01 to 2 weight %, preferably 0.1 to 0.5 weight %, and the hydroxyphosphono carboxylic acid in an amount of 1 to 5 weight %, preferably 2 to 5 weight %.

10. Additive according to claim 5, **characterised in that** the hydroxyphosphono carboxylic acid is bonded to the glycerine or to a fatty acid esterified with the glycerine.

11. Additive for a glycol and/or a glycol ether exhibiting:
a compound exhibiting a sterically inhibited hydroxyphenyl group,
a hydroxyphosphono carboxylic acid and
an aromatic aminic anti-oxidant.

12. Additive according to claim 11, **characterised in that** the compound exhibiting a sterically inhibited hydroxyphenyl group and the hydroxyphosphono carboxylic acid are bonded to each other.

13. Additive according to claim 11, **characterised in that** the compound exhibiting a sterically inhibited hydroxyphenyl group is a glycerine ester with at least one linear carboxylic acid exhibiting the sterically inhibited hydroxyphenyl group, wherein the hydroxyphenyl group is bonded in para position to the carboxylic acid esterified with the glycerine.

14. Additive according to claim 13, **characterised in that** the linear carboxylic acid exhibiting the sterically inhibited hydroxyphenyl group exhibits a chain of 8 to 18 carbon atoms.

15. Additive according to any one of claims 11 through 14, **characterised in that** the hydroxyphosphono carboxylic acid exhibits the following structure 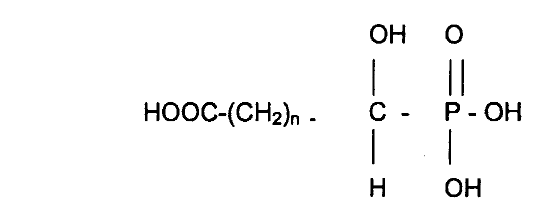 where n = 0 .... 8.

16. Additive according to claim 11, **characterised in that** it contains at least 70 weight % triethylene glycol, the compound exhibiting a sterically inhibited hydroxyphenyl group in an amount of 0.01 to 2 weight %, preferably 0.1 to 0.5 weight %, the hydroxyphosphono carboxylic acid in an amount of 1 to 5 weight %, preferably 2 to 5 weight %, and the aromatic aminic anti-oxidant in an amount of 0.01 to 2 weight %, preferably 0.1 to 0.5 weight %.

17. Additive according to claim 11, **characterised in that** it contains a polycarboxylic acid.

18. Additive according to claim 17, **characterised in that** it contains a polycarboxylic acid containing nitrogen.

19. Additive according to claim 18, **characterised in that** the polycarboxylic acid contains at least two carboxylic acids bonded via imino groups to a derivative of triazine.

20. Additive according to claim 16, **characterised in that** it contains a polycarboxylic acid in an amount of 1 to 10 weight %, preferably 2.5 to 5 weight %.

21. Additive according to claim 20, **characterised in that** the polycarboxylic acid exhibits 6,6',6"-(1,3,5-triazine-2,4,6-triyltriimino)-trihexanoic acid.

22. Additive according to claim 11, **characterised in that** it contains an alkanolamine.

23. Additive according to claim 22, **characterised in that** the alkanolamine is a tertiary alkanolamine.

24. Additive for a glycol or a glycol ether exhibiting:
a hydroxyphosphono carboxylic acid and
a tertiary alkanolamine.

25. Additive according to claim 24, **characterised in that** the hydroxyphosphono carboxylic acid has the following structure 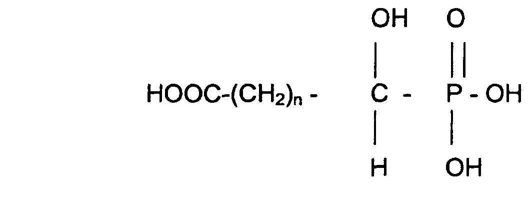 where n = 0 ... 8.

26. Additive according to claim 24, **characterised in that** the tertiary alkanolamine comprises triethanolamine and/or methyldiethanolamine.

27. Additive according to claim 24, **characterised in that** it contains a polycarboxylic acid.

28. Additive according to claim 27, **characterised in that** it contains a polycarboxylic acid containing nitrogen.

29. Additive according to claim 28, **characterised in that** the polycarboxylic acid contains at least two carboxylic acids bonded via imino groups to a derivative of triazine.

## Revendications

1. Utilisation d'un phosphonate organique comme agent anti-vieillissement résistant aux températures pour un glycol circulant dans une installation de déshydratation de gaz, ledit glycol étant utilisé dans un procédé dans lequel les températures, à large exemption d'oxygène, atteignent jusqu'à environ 220 °C, le phosphonate étant ajouté à une quantité entre 0,01 et 0,1 % en poids, de préférence de 0,02 à 0,05 % en poids.

2. Procédé suivant la revendication 1, **caractérisé par le fait que** le phosphonate est mélangé, avant son utilisation comme additif dans le procédé, à un glycol, de préférence triéthylèneglycol, à une quantité entre 1 et 5 % en poids, de préférence de 2 à 5 % en poids, et que ce mélange est ajouté, à une concentration d'environ 1 % en poids, au glycol circulant dans l'installation de déshydratation de gaz.

3. Procédé suivant la revendication 1, **caractérisé par le fait que** le phosphonate utilisé est un acide d'hydroxy-phosphono-carbone.

4. Procédé suivant la revendication 3, **caractérisé par le fait que** qu'un acide d'hydroxy-phosphono-carbone, présentant la structure suivante est utilisé : 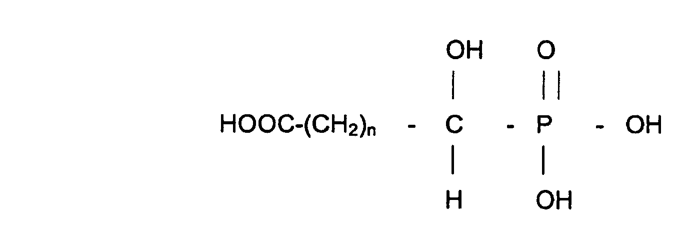 où : n = 0 .... 8.

5. Additif pour un glycol ou un glycolether présentant :
un ester du glycerine avec au moins un acide de carbone linéaire contenant un hydroxyphényl stériquement empêché, et
un acide d'hydroxy-phosphono-carbone.

6. Additif suivant la revendication 5, **caractérisé par le fait que** l'ester est un ester de mélange partiel du glycerine avec au moins un acide de carbone linéaire contenant un hydroxyphényl stériquement empêché et au moins un autre acide de carbone linéaire.

7. Additif suivant la revendication 5, **caractérisé par le fait que** l'hydroxyphényl stériquement empêché est lié, en position para, à l'acide de carbone estérifié avec le glycerine.

8. Additif suivant la revendication 5, **caractérisé par le fait que** l'acide de carbone linéaire présentant l'hydroxyphényl stériquement empêché présente une chaîne de 8 à 18 atomes de carbone.

9. Additif suivant la revendication 6, **caractérisé par le fait qu'**il contient au moins 70 % en poids de triéthylèneglycol, l'ester de mélange partiel du glycerine à une quantité de 0,01 à 2 % en poids, de préférence de 0,1 à 0,5 % en poids, et l'acide d'hydroxy-phosphono-carbone à une quantité de 1 à 5 % en poids, de préférence de 2 à 5 % en poids.

10. Additif suivant la revendication 5, **caractérisé par le fait que** l'acide d'hydroxyphosphono-carbone est lié au glycerine ou à un acide gras estérifié avec le glycerine.

11. Additif pour un glycol et/ou un glycolether présentant :
une combinaison présentant un hydroxyphényl stériquement empêché,
un acide d'hydroxy-phosphono-carbone, et
un antioxydant aminé aromatique.

12. Additif suivant la revendication 11, **caractérisé par le fait que** la combinaison présentant un hydroxyphényl stériquement empêché et l'acide d'hydroxyphosphono-carbone sont liés l'une à l'autre.

13. Additif suivant la revendication 11, **caractérisé par le fait que** la combinaison présentant un hydroxyphényl stériquement empêché est un ester du glycerine avec au moins un acide de carbone linéaire contenant l'hydroxyphényl stériquement empêché, l'hydroxyphényl étant lié, en position para, à l'acide de carbone estérifié avec le glycerine.

14. Additif suivant la revendication 13, **caractérisé par le fait que** l'acide de carbone linéaire présentant l'hydroxyphényl stériquement empêché présente une chaîne de 8 à 18 atomes de carbone.

15. Additif suivant l'une des revendications 11 à 14, **caractérisé par le fait que** l'acide d'hydroxy-phosphono-carbone présente la structure suivante : 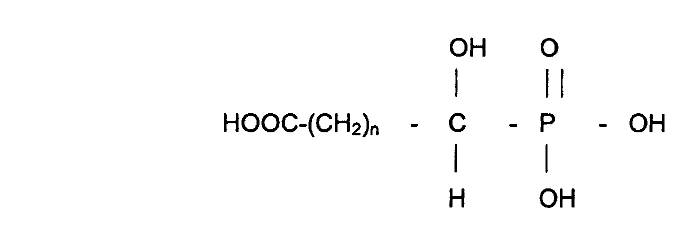 où : n = 0 .... 8.

16. Additif suivant la revendication 11, **caractérisé par le fait qu'**il contient au moins 70 % en poids de triéthylèneglycol, la combinaison présentant un hydroxyphényl stériquement empêché à une quantité de 0,01 à 2 % en poids, de préférence de 0,1 à 0,5 % en poids, l'acide d'hydroxy-phosphono-carbone à une quantité de 1 à 5 % en poids, de préférence de 2 à 5 % en poids, et l'antioxydant aminé aromatique à une quantité de 0,01 à 2 % en poids, de préférence de 0,1 à 0,5 % en poids.

17. Additif suivant la revendication 11, **caractérisé par le fait qu'**il contient un acide polycarbone.

18. Additif suivant la revendication 17, **caractérisé par le fait qu'**il contient un acide polycarbone azoté.

19. Additif suivant la revendication 18, **caractérisé par le fait que** l'acide polycarbone contient au moins deux acides de carbone liés par des groupes iminogènes à un dérivé de la triazine.

20. Additif suivant la revendication 16, **caractérisé par le fait qu'**il contient un acide polycarbone à une quantité de 1 à 10 % en poids, de préférence de 2,5 à 5 % en poids.

21. Additif suivant la revendication 20, **caractérisé par le fait que** l'acide polycarbone contient l'acide 6,6',6"-(1,3,5-triazine-2,4,6-triyltriimino)-trihexane.

22. Additif suivant la revendication 11, **caractérisé par le fait qu'**il contient une alcanolamine.

23. Additif suivant la revendication 22, **caractérisé par le fait que** l'alcanolamine est une alcanolamine tertiaire.

24. Additif pour un glycol ou un glycolether présentant :
un acide d'hydroxy-phosphono-carbone, et
une alcanolamine tertiaire.

25. Additif suivant la revendication 24, **caractérisé par le fait que** l'acide d'hydroxyphosphono-carbone présente la structure suivante : 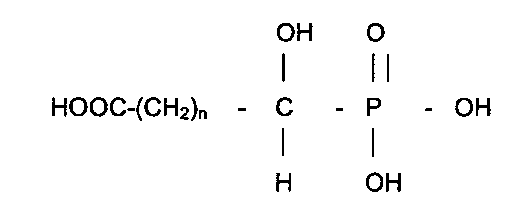 où : n = 0 .... 8.

26. Additif suivant la revendication 24, **caractérisé par le fait que** l'alcanolamine tertiaire comprend de triethanolamine et/ou de methyldiethanolamine.

27. Additif suivant la revendication 24, **caractérisé par le fait qu'**il contient un acide polycarbone.

28. Additif suivant la revendication 27, **caractérisé par le fait qu'**il contient un acide polycarbone azoté.

29. Additif suivant la revendication 28, **caractérisé par le fait que** l'acide polycarbone contient au moins deux acides de carbone liés par des groupes iminogènes à un dérivé de la triazine.
